# EUROPÄISCHE PATENTSCHRIFT

(11) **EP 3 023 065 B1**
(45) Veröffentlichungstag und Bekanntmachung des Hinweises auf die Patenterteilung: **04.11.2020**
(21) Anmeldenummer: 15195764.4
(22) Anmeldetag: 23.11.2015
(51) Int. Cl.: A61B 17/70

(54) **PEDIKELSCHRAUBENSYSTEM UND WIRBELSÄULENSTABILISIERUNGSSYSTEM**
PEDICLE SCREW SYSTEM AND SPINAL COLUMN-STABILIZING SYSTEM
SYSTÈME DE VIS PÉDICULAIRE ET SYSTÈME DE STABILISATION DE COLONNE VERTÉBRALE

(30) Priorität: 24.11.2014 DE 102014117176
(43) Veröffentlichungstag der Anmeldung: 25.05.2016
(73) Patentinhaber: AESCULAP AG, 78532 Tuttlingen (DE)
(72) Erfinder: Beger, Jens, 78532 Tuttlingen (DE); Stoerk, Claudia, 78576 Emmingen (DE); Krüger, Sven, 78647 Trossingen (DE)
(74) Vertreter: Hoeger, Stellrecht & Partner Patentanwälte mbB

(56) Entgegenhaltungen:
- DE-A1-102011 053 295
- DE-U1- 9 202 587
- US-A1- 2008 262 318
- US-A1- 2015 238 235

## Beschreibung

Die vorliegende Erfindung betrifft ein Pedikelschraubensystem umfassend eine Pedikelschraube mit einem Schraubenschaft, welcher ein Außengewinde aufweist, und einem kugelgelenkig am Schraubenschaft gelagerten Schraubenkopf, welcher Schraubenkopf eine Verbindungselementaufnahme für ein Verbindungselement eines Wirbelsäulenstabilisierungssystems umfasst.

Ferner betrifft die vorliegende Erfindung ein Wirbelsäulenstabilisierungssystem umfassend mindestens zwei Knochenschrauben und mindestens ein an den mindestens zwei Knochenschrauben festlegbares Verbindungselement.

Pedikelschrauben und Wirbelsäulenstabilisierungssysteme der eingangs beschriebenen Art sind beispielsweise aus der DE 10 2013 100 574 A1 bekannt. Mit ihnen lassen sich beispielsweise bei Deformitätenoperationen deformierte Wirbelsäulen durch entsprechende Implantation und Ausrichtung von Pedikelschrauben in eine gewünschte Form bringen und in dieser fixieren. Zur Ausrichtung einzelner, fehlgestellter Wirbel werden die Kräfte für die Korrekturmanöver über die Pedikelschrauben in den jeweiligen Wirbel eingeleitet.

Bei Pedikelschraubensystemen, bei denen ein Verbindungselement von oben in eine entsprechende Verbindungselementaufnahme am Schraubenkopf eingesetzt werden kann, also bei sogenannten "Tulpen"-Systemen, ist eine Krafteinleitung nicht möglich, wenn die Pedikelschraube in Form einer Polyaxialschraube ausgebildet ist. Eine Krafteinleitung ist nur möglich, wenn der Schraubenkopf relativ zum Schraubenschaft unbeweglich oder maximal um eine Achse verschwenkbar ist, es sich also bei der Pedikelschraube um eine sogenannte Monoaxialschraube handelt. Der Schraubenkopf wird dabei in einer Ebene bewegt, welche senkrecht zur Achse, um die verschwenkt wird, verläuft, so dass die Monoaxialschraube in diesem Sinne auch als Uniplanarschraube bezeichnet werden kann. Bei Polyaxialschrauben hingegen, die das Einsetzen des Verbindungselements, beispielsweise eines Stabs, deutlich dadurch vereinfachen, dass der Schraubenkopf beliebig relativ zum Schraubenschaft orientiert werden kann, ist eine solche Krafteinleitung und Korrektur einer Ausrichtung eines Wirbels nicht oder nur rudimentär möglich. Insbesondere ist es nicht möglich, die Technik der segmentalen Derotation bei Polyaxialschrauben anzuwenden. Diese ist nur bei direkter Krafteinleitung in die Pedikelschraube, wie es insbesondere die beschriebenen Monoaxialschrauben ermöglichen, realisierbar.

Aus der DE 10 2011 053 295 A1 ist eine polyaxiale Pedikelschraube mit provisorischer Fixierung bekannt. In der DE 92 02 587 U1 ist ein Repositions-Instrument beschrieben. Eine Retraktionsvorrichtung zum minimalinvasiven Öffnen eines Zwischenwirbelzugangs ist in der US 2008/0262318 offenbart. Außerdem ist aus der US 2015/0238235 A1 ein Instrument zum Verriegeln und Verhindern einer Rotation einer polyaxialen Pedikelschraube bekannt.

Es ist daher eine Aufgabe der vorliegenden Erfindung, ein Pedikelschraubensystem und ein Wirbelsäulenstabilisierungssystem der eingangs beschriebenen Art so weiter zu bilden, dass deren Handhabung verbessert wird.

Diese Aufgabe wird bei einem Pedikelschraubensystem der eingangs beschriebenen Art erfindungsgemäß dadurch gelöst, dass es eine Knochenausrichtvorrichtung und eine Kopplungseinrichtung umfasst zum kraft- und/oder formschlüssigen Koppeln der Knochenausrichtvorrichtung und der Pedikelschraube in einer Ausrichtstellung, in welcher eine Beweglichkeit des Schraubenkopfs und des Schraubenschafts von drei Bewegungsfreiheitsgraden der Rotation des kugelgelenkig am Schraubenschaft gelagerten Schraubenkopfs um mindestens einen Rotationsfreiheitsgrad reduziert ist, nämlich von ursprünglich drei Bewegungsfreiheitsgraden der Rotation auf einen oder zwei Bewegungsfreiheitsgrade der Rotation, wobei der Schraubenkopf eine in Richtung auf das Außengewinde hin weisende Anlagefläche aufweist, an welcher sich die Knochenausrichtvorrichtung in der Ausrichtstellung abstützt.

Die erfindungsgemäß vorgeschlagene Weiterbildung ermöglicht es dem Operateur insbesondere, über die Knochenausrichtvorrichtung Kräfte zum Bewegen, insbesondere Rotieren, deformierter Wirbel einer Wirbelsäule direkt auf den Schraubenschaft einzuleiten, wenn die Kopplungseinrichtung des Pedikelschraubensystems die Ausrichtstellung einnimmt. Das vorgeschlagene Pedikelschraubensystem vereinigt somit einerseits die Vorteile von Polyaxialschrauben, die eine beliebige Ausrichtung des Schraubenkopfs relativ zum Schraubenschaft ermöglichen, um das Einsetzen des Verbindungselements in die Verbindungselementaufnahme am Schraubenkopf zu erleichtern, und andererseits, insbesondere bei der Beschränkung der Bewegungsfreiheitsgrade der Rotation auf einen Rotationsfreiheitsgrad, die Vorteile einer Monoaxialschraube, die das Einleiten von Kräften auf den Schraubenschaft zur Ausrichtung eines Wirbelkörpers, in den die Pedikelschraube eingeschraubt ist, ermöglicht. Die Zahl der ursprünglich drei Bewegungsfreiheitsgrade der Rotation kann auf einen oder zwei Bewegungsfreiheitsgrade der Rotation beschränkt sein. Gemäß der Erfindung weist der Schraubenkopf eine in Richtung auf das Außengewinde hin weisende Anlagefläche auf, an welcher sich die Knochenausrichtvorrichtung in der Ausrichtstellung abstützt. Diese Ausgestaltung ermöglicht insbesondere eine direkte Kraftübertragung von der Knochenausrichtvorrichtung auf den Schraubenkopf beziehungsweise eine direkte Einwirkung auf diesen zum Blockieren von mindestens zwei Rotationsfreiheitsgraden des kugelgelenkig mit dem Schraubenschaft zusammenwirkenden Schraubenkopfs.

Durch die Kopplung der Knochenausrichtvorrichtung und der Pedikelschraube in der Ausrichtstellung wird insbesondere temporär eine Anordnung geschaffen, die in ihrer Funktion einer Monoaxialschraube entspricht. Von den ursprünglich drei Bewegungsfreiheitsgraden, die die kugelgelenkige Verbindung zwischen dem Schraubenschaft und dem Schraubenkopf ermöglicht, wird durch die Kopplung der Pedikelschraube und der Knochenausrichtvorrichtung in der Ausrichtstellung die Zahl der Bewegungsfreiheitsgrade zwischen Schraubenschaft und Schraubenkopf auf einen Bewegungsfreiheitsgrad der Rotation reduziert. Dies entspricht einer Rotation des Schraubenschafts und des Schraubenkopfs relativ zueinander um lediglich eine einzige Schwenkachse. Durch die Kopplungseinrichtung kann insbesondere eine axiale und/oder drehfeste Verbindung zwischen der Knochenausrichtvorrichtung und der Pedikelschraube hergestellt und somit über die Knochenausrichtvorrichtung eine indirekte Kraftübertragung beispielsweise von der Knochenausrichtvorrichtung auf den Schraubenschaft ermöglicht werden.

Günstig ist es, wenn die Knochenausrichtvorrichtung in Form einer Klemmplatte ausgebildet ist oder eine Klemmplatte umfasst. Beispielsweise kann die Klemmplatte an der Pedikelschraube zwischen dem Schraubenkopf und dem Außengewinde klemmend angeordnet werden, um so eine freie Rotation des Schraubenkopfs relativ zum Schraubenschaft teilweise zu blockieren und nur noch eine Verschwenkbewegung um eine einzige Schwenkachse, wie dies bei einer Monoaxialschraube der Fall ist, zu gestatten.

Vorteilhaft ist es, wenn die Kopplungseinrichtung erste und zweite Kopplungselemente umfasst, welche in der Ausrichtstellung kraft- und/oder formschlüssig in Eingriff stehen und einerseits an der Knochenausrichtvorrichtung und andererseits an der Pedikelschraube angeordnet oder ausgebildet sind. Mit einer solchen Kopplungseinrichtung kann die Knochenausrichtvorrichtung mit der Pedikelschraube auf einfache und sichere Weise in Eingriff gebracht werden.

Vorzugsweise sind das erste Kopplungselement in Form eines Kopplungsvorsprungs und das zweite Kopplungselement in Form einer zum Kopplungsvorsprung korrespondierenden Kopplungsaufnahme ausgebildet. Derartige Kopplungselemente lassen sich auf einfache Weise ausbilden und wahlweise an der Pedikelschraube oder an der Knochenausrichtvorrichtung anordnen oder ausbilden.

Um insbesondere ein unbeabsichtigtes Ablösen der Knochenausrichtvorrichtung von der Pedikelschraube in der Ausrichtstellung zu vermeiden oder ein Risiko hierfür zu minimieren, ist es vorteilhaft, wenn der Kopplungsvorsprung in der Ausrichtstellung formschlüssig oder im Wesentlichen formschlüssig in die Kopplungsaufnahme eingreift.

Ein besonders stabiles Pedikelschraubensystem lässt sich insbesondere dadurch erhalten, dass der Kopplungsvorsprung einstückig mit dem Schraubenschaft ausgebildet ist.

Auf besonders einfache Weise herstellen lässt sich das Pedikelschraubensystem, wenn der Kopplungsvorsprung in Form eines gewindefreien Schaftabschnitts des Schraubenschafts ausgebildet ist. Der gewindefreie Schaftabschnitt kann insbesondere einen kreisförmigen Querschnitt aufweisen oder einen unrunden Querschnitt, beispielsweise in Form eines Ovals, eines Vielrunds oder eines Vielecks. So kann beispielsweise bei einem kreisförmigen Querschnitt eine Verdrehung zwischen der Knochenausrichtvorrichtung und der Pedikelschraube ermöglicht werden oder aber auch eine drehfeste Verbindung mit einem unrunden Querschnitt hergestellt werden.

Gemäß einer weiteren bevorzugten Ausführungsform der Erfindung kann vorgesehen sein, dass der Schraubenschaft einen Gelenkkopf aufweist und dass der Schraubenkopf eine zum Gelenkkopf korrespondierende Gelenkkopfaufnahme zur Ausbildung eines Kugelgelenks im Zusammenwirken mit dem Gelenkkopf aufweist. Durch den Gelenkkopf im Zusammenwirken mit der Gelenkkopfaufnahme kann so auf einfache Weise ein Kugelgelenk gebildet werden. Der Gelenkkopf kann insbesondere die Form einer Kugel oder eines Teils einer Kugel aufweisen. Die Gelenkkopfaufnahme kann insbesondere in Form eines zu einer Gelenkkugel korrespondierenden hohlkugeligen Sitzes geformt sein.

Auf einfache Weise lassen sich Rotationsfreiheitsgrade zwischen dem Schraubenkopf und dem Schraubenschaft ausschalten, wenn der Kopplungsvorsprung zwischen dem Außengewinde und dem Gelenkkopf angeordnet ist. Beispielsweise kann so mit der Knochenausrichtvorrichtung eine Bewegung des Schraubenkopfs relativ zum Schraubenschaft auf einfache Weise blockiert werden, beispielsweise durch eine klemmende Verbindung.

Vorteilhaft ist es, wenn die Kopplungseinrichtung ein Anschlagelement umfasst, an welchem sich die Knochenausrichtvorrichtung in der Ausrichtstellung abstützt. Beispielsweise kann das Anschlagelement am Schraubenschaft ausgebildet sein, sodass sich die Knochenausrichtvorrichtung einerseits am Anschlagelement und andererseits am Schraubenkopf abstützen kann.

Günstigerweise weist die Knochenausrichtvorrichtung eine Anschlagelementanlagefläche auf, welche in der Ausrichtstellung am Anschlagelement anliegt. Dies ermöglicht es insbesondere, dass sich die Knochenausrichtvorrichtung in der Ausrichtstellung direkt am Anschlagelement abstützt. Wenn sich die Knochenausrichtvorrichtung andererseits am Schraubenkopf abstützen kann, kann eine Beweglichkeit des Schraubenkopfs relativ zum Schraubenschaft auf einfache Weise eingeschränkt werden.

Vorzugsweise ist die Anschlagelementanlagefläche eben oder im Wesentlichen eben ausgebildet. Diese Ausgestaltung ermöglicht insbesondere eine flächige Anlage am Anschlagelement, wenn dieses eine ebene oder im Wesentlichen ebene Anschlagfläche aufweist, an welcher die Anschlagelementanlagefläche in der Ausrichtstellung anliegt.

Günstig ist es, wenn das Anschlagelement eine ebene oder im Wesentlichen ebene Anschlagfläche aufweist, an welcher sich die Knochenausrichtvorrichtung in der Ausrichtstellung abstützt. Eine solche Anschlagfläche ist auf einfache Weise auszubilden und ermöglicht eine flächige Anlage der Knochenausrichtvorrichtung am Anschlagelement, wenn die Anschlagelementanlagefläche eben oder im Wesentlichen eben ausgebildet ist.

Ein besonders kompakter Aufbau des Pedikelschraubensystems lässt sich insbesondere dadurch erreichen, dass das Anschlagelement am Schraubenschaft angeordnet oder ausgebildet ist.

Besonders einfach herstellen lässt sich der Schraubenschaft, wenn das Anschlagelement in Form eines Ringflansches ausgebildet ist. Beispielsweise kann der Schraubenschaft so durch Gießen oder spanende Bearbeitung hergestellt werden.

Gemäß einer weiteren bevorzugten Ausführungsform der Erfindung kann vorgesehen sein, dass das Anschlagelement einstückig mit dem Schraubenschaft ausgebildet oder kraft- und/oder stoffschlüssig mit dem Schraubenschaft verbunden ist. Insbesondere kann das Anschlagelement mit dem Schraubenschaft verpresst, verklebt, verlötet oder verschweißt sein. Je nach Art und Ausbildung des Schraubenschafts und des Schraubenkopfs sowie der Art der kugelgelenkigen Verbindung derselben kann es wahlweise vorteilhaft sein, das Anschlagelement einstückig mit dem Schraubenschaft auszubilden oder kraft- und/oder stoffschlüssig mit diesem zu verbinden.

Vorteilhaft ist es, wenn ein Außendurchmesser des Anschlagelements größer ist als ein maximaler Außendurchmesser des Außengewindes. So kann sichergestellt werden, dass in der Ausrichtstellung die Knochenausrichtvorrichtung nicht mit dem Außengewinde in Kontakt treten kann.

Insbesondere bei kurzen Schraubenschäften ist es vorteilhaft, wenn das Anschlagelement direkt an das Außengewinde angrenzt. So kann der Schraubenschaft bis zum Anschlagelement in einen Knochen eingeschraubt werden.

Auf besonders einfache Weise herstellen lässt sich der Schraubenkopf, wenn die Anlagefläche eben oder im Wesentlichen eben oder vom Schraubenkopf weg weisend konkav gekrümmt ausgebildet ist. Vorzugsweise wird die Anlagefläche derart geformt, dass eine flächige Anlage an der Knochenausrichtvorrichtung möglich ist.

Gemäß einer weiteren bevorzugten Ausführungsform der Erfindung kann vorgesehen sein, dass die Knochenausrichtvorrichtung eine Führungsfläche zum Vorgeben einer Bewegungsrichtung für eine Relativbewegung zwischen dem Schraubenkopf und dem Schraubenschaft aufweist und dass der Schraubenkopf in der Ausrichtstellung an der Führungsfläche anliegt und an dieser um eine Schwenkachse relativ zum Schraubenschaft verschwenkbar geführt ist. Mit der Führungsfläche ist es so insbesondere auf einfache Weise möglich, die gewünschte Relativbewegung zwischen dem Schraubenkopf und dem Schraubenschaft vorzugeben, also insbesondere nur eine Verschwenkbewegung um eine einzige Schwenkachse zuzulassen. Dies kann auf einfache Weise durch Formgebung der Führungsfläche erreicht werden. Insbesondere kann die Führungsfläche in Richtung auf den Schraubenkopf hin weisend konvex gekrümmt sein. Beispielsweise kann sie sich koaxial zur Schwenkachse erstrecken oder kann die Schwenkachse auf der Führungsfläche liegen.

Vorzugsweise bildet die Führungsfläche einen Teil einer Zylinderoberfläche. Günstigerweise bildet sie einen Teil einer Oberfläche eines geraden Kreiszylinders. Insbesondere kann eine vom geraden Kreiszylinder definierte Längsachse die Schwenkachse definieren oder kann die Schwenkachse auf der Oberfläche liegen und parallel zur Längsachse des geraden Kreiszylinders verlaufen.

Vorteilhafterweise verläuft die Schwenkachse in der Ausrichtstellung quer zu einer Schraubenschaftlängsachse des Schraubenschafts. Insbesondere kann die Schwenkachse senkrecht zur Schraubenschaftlängsachse verlaufen. Selbstverständlich kann die Schwenkachse auch bezogen auf eine senkrecht zur Schraubenschaftlängsachse verlaufende Ebene geneigt verlaufen und mit dieser einen Neigungswinkel einschließen, beispielsweise in einem Bereich von etwa 0° bis etwa 30°. Dies ermöglicht es insbesondere, den Schraubenkopf und den Schraubenschaft in der Ausrichtstellung relativ zueinander um den Neigungswinkel geneigt zu halten, jedoch noch eine Verschwenkbewegung um die Schwenkachse zu ermöglichen.

Vorteilhaft ist es, wenn die Knochenausrichtvorrichtung einen Führungskörper umfasst und wenn der Führungskörper die Führungsfläche, die Anschlagelementanlagefläche und/oder eines der Kopplungselemente der Kopplungseinrichtung umfasst. Ein derart ausgebildeter Führungskörper kann somit mehrere Funktionen ausüben und ermöglicht insgesamt einen möglichst kompakten Aufbau der Knochenausrichtvorrichtung und des Pedikelschraubensystems.

Vorzugsweise ist die Kopplungsaufnahme in Form einer Ausnehmung oder Durchbrechung der Knochenausrichtvorrichtung ausgebildet. Diese lassen sich einfach herstellen.

Günstigerweise ist die Kopplungsaufnahme in Form einer Bohrung ausgebildet. Eine solche ist auf einfache Weise herzustellen. Insbesondere kann eine Längsachse der Bohrung auch eine Ausrichtung des Schraubenkopfs relativ zum Schraubenschaft vorgeben.

Um die Handhabung des Pedikelschraubensystems für einen Operateur weiter zu verbessern, ist es günstig, wenn die Knochenausrichtvorrichtung einen Kopplungsabschnitt und einen Handhabungsabschnitt umfasst und wenn der Kopplungsabschnitt eines der Kopplungselemente der Kopplungseinrichtung umfasst. Mit einer solchen Knochenausrichtvorrichtung ist es möglich, eine Verbindung in der Ausrichtstellung zwischen dem Kopplungsabschnitt und der Pedikelschraube herzustellen. Ein Operateur kann dann den Handhabungsabschnitt entweder mit einer Hand oder einem weiteren Instrument fassen und auf diese Weise eine Kraft auf die Pedikelschraube ausüben zum Ausrichten des Schraubenschafts und damit des mit diesem verbundenen Wirbels in einer gewünschten Weise.

Auf besonders einfache Weise lässt sich die Knochenausrichtvorrichtung mit der Pedikelschraube koppeln, wenn die Knochenausrichtvorrichtung auf die Pedikelschraube aufclipsbar oder aufrastbar ausgebildet ist. Vorzugsweise ist der Kopplungsabschnitt der Knochenausrichtvorrichtung auf die Pedikelschraube aufclipsbar oder aufrastbar ausgebildet.

Eine einfache Kopplung zwischen der Knochenausrichtvorrichtung und der Pedikelschraube kann insbesondere dadurch realisiert werden, dass der Kopplungsabschnitt zwei im Wesentlichen parallel zueinander verlaufende, aus einer Grundstellung federnd aufeinander zu oder voneinander weg bewegbare Klemmschenkel aufweist und dass die Klemmschenkel durch einen Einführschlitz zum Einführen des an der Pedikelschraube ausgebildeten Kopplungselements voneinander getrennt sind. Beispielsweise kann das an der Pedikelschraube ausgebildete Kopplungselement in Form eines gewindefreien Schaftabschnitts in den Einführschlitz eingeführt werden, sodass dieser etwas ausgeweitet wird, bis das Kopplungselement der Pedikelschraube in die Kopplungselementaufnahme der Knochenausrichtvorrichtung in der Ausrichtstellung eingreift, beispielsweise einschnappt. Die Klemmschenkel können dann in der Ausrichtstellung wieder in ihre Grundstellung zurückfedern oder in der Ausrichtstellung noch etwas unter Vorspannung gehalten sein, sodass eine zusätzliche Klemmwirkung der Klemmschenkel erreicht wird, um die Knochenausrichtvorrichtung klemmend an der Pedikelschraube zu halten.

Um das Einführen des an der Pedikelschraube ausgebildeten Kopplungselements in die Kopplungsaufnahme der Knochenausrichtvorrichtung zu erleichtern, ist es vorteilhaft, wenn eine Breite des Einführschlitzes in Richtung auf ein freies Ende der Klemmschenkel hin zunimmt. Beispielsweise können freie Enden der Klemmschenkel Aufgleitflächen aufweisen, die das Einführen des an der Pedikelschraube ausgebildeten Kopplungselements in den Einführschlitz zu erleichtern.

Damit das in Eingriff Bringen der Knochenausrichtvorrichtung mit der Pedikelschraube weiter erleichtert wird, ist es günstig, wenn sich der Einführschlitz teilweise in den Handhabungsabschnitt hinein erstreckt. Dies ermöglicht insbesondere die Ausbildung sehr langer Klemmschenkel, die dann zum Koppeln der Knochenausrichtvorrichtung und der Pedikelschraube miteinander in der Ausrichtstellung nur wenig aufgespreizt werden müssen.

Um die Knochenausrichtvorrichtung im Wesentlichen ohne die Ausübung von Klemmkräften mit der Pedikelschraube zu koppeln, ist es vorteilhaft, wenn ein Innendurchmesser der Kopplungsaufnahme an einen Außendurchmesser des gewindefreien Schaftabschnitts angepasst ist.

Ferner kann vorgesehen sein, dass die Kopplungsaufnahme eine Kopplungsaufnahmelängsachse definiert und dass die Kopplungsaufnahmelängsachse quer zur Schwenkachse verläuft. Beispielsweise kann sie senkrecht zur Schwenkachse verlaufen. Insbesondere kann die Kopplungsaufnahme derart ausgebildet sein, dass die Kopplungsaufnahmelängsachse in der Ausrichtstellung mit einer vom Schraubenkopf definierten Längsachse zusammenfällt und relativ zur Schraubenschaftlängsachse um den oben beschriebenen Neigungswinkel geneigt ist.

Um insbesondere einen Einsatz des Pedikelschraubensystems bei minimalinvasiven chirurgischen Eingriffen zu ermöglichen, ist es vorteilhaft, wenn der Kopplungsabschnitt und der Handhabungsabschnitt gegeneinander abgewinkelt sind. Vorzugsweise liegt ein Winkel der Abwinklung in einem Bereich von etwa 70° bis etwa 110°.

Damit die Knochenausrichtvorrichtung einfach und sicher gefasst und gehalten werden kann, ist es vorteilhaft, wenn der Handhabungsabschnitt einen die beiden Klemmschenkel miteinander verbindenden Griffbereich umfasst. So kann ein Operateur die Knochenausrichtvorrichtung mit der daran gekoppelten Pedikelschraube einfach und sicher manipulieren.

Ferner ist es günstig, wenn das Pedikelschraubensystem mehrere Knochenausrichtvorrichtungen umfasst, bei denen die Anschlagelementanlagefläche und eine Führungsflächenlängsachse der Führungsfläche unterschiedliche Ausrichtwinkel relativ zueinander aufweisen. Mit einem solchen Satz von Knochenausrichtvorrichtungen des Pedikelschraubensystems kann ein Operateur jeweils diejenige Knochenausrichtvorrichtung auswählen, mit der er einen festen Neigungswinkel des Schraubenschafts und des Schraubenkopfs relativ zueinander in der Ausrichtstellung vorgeben möchte. Beispielsweise kann der Satz um jeweils 10° abgestufte Neigungswinkel aufweisen, um den Neigungswinkel des Schraubenschafts und des Schraubenkopfs relativ zueinander in der Ausrichtstellung in definierten Stufen vorgeben zu können, beispielsweise mit Neigungswinkeln von 0°, 10°, 20° und so weiter.

Vorteilhaft ist es, wenn die Knochenausrichtvorrichtung eine Schraubenschaftaufnahme aufweist, in die der Schraubenschaft in der Ausrichtstellung mindestens teilweise eingreift. Insbesondere kann der Schraubenschaft die Schraubenschaftaufnahme in der Ausrichtstellung durchsetzen, also beispielsweise mit einem distalen Ende des Schraubenschafts aus der Schraubenschaftaufnahme distalseitig vorstehen.

Besonders einfach und kompakt ausbilden lässt sich das Pedikelschraubensystem, wenn die Kopplungsaufnahme die Schraubenschaftaufnahme definiert.

Die eingangs gestellte Aufgabe wird ferner bei einem Wirbelsäulenstabilisierungssystem der eingangs beschriebenen Art erfindungsgemäß dadurch gelöst, dass mindestens eine der mindestens zwei Knochenschrauben in Form eines der oben beschriebenen Pedikelschraubensysteme ausgebildet ist.

Ein derart weitergebildetes Wirbelsäulenstabilisierungssystem weist dann insbesondere auch die oben im Zusammenhang mit bevorzugten Ausführungsformen von Pedikelschraubensystemen beschriebenen Vorteile auf.

Die nachfolgende Beschreibung einer bevorzugten Ausführungsform der Erfindung dient im Zusammenhang mit der Zeichnung der näheren Erläuterung. Es zeigen:
- Figur 1:: eine schematische Ansicht eines Wirbelsäulenstabilisierungssystems umfassend zwei Knochenschrauben und ein Verbindungselement, welches an einer Wirbelsäule festgelegt ist;
- Figur 2:: eine Seitenansicht einer in einen Wirbel eingeschraubten Pedikelschraube mit daran gekoppelter Knochenausrichtvorrichtung;
- Figur 3:: eine weitere Seitenansicht des in Figur 2 dargestellten Pedikelschraubensystems;
- Figur 4:: eine perspektivische Ansicht des Pedikelschraubensystems aus Figur 2;
- Figur 5:: eine Ansicht der Anordnung aus Figur 4 von vorn;
- Figur 6:: eine Seitenansicht einer mit einer Pedikelschraube gekoppelten Knochenausrichtvorrichtung;
- Figur 7:: eine Ansicht in Richtung des Pfeils A in Figur 6; und
- Figur 8:: eine perspektivische Ansicht der Knochenausrichtvorrichtung aus Figur 6.

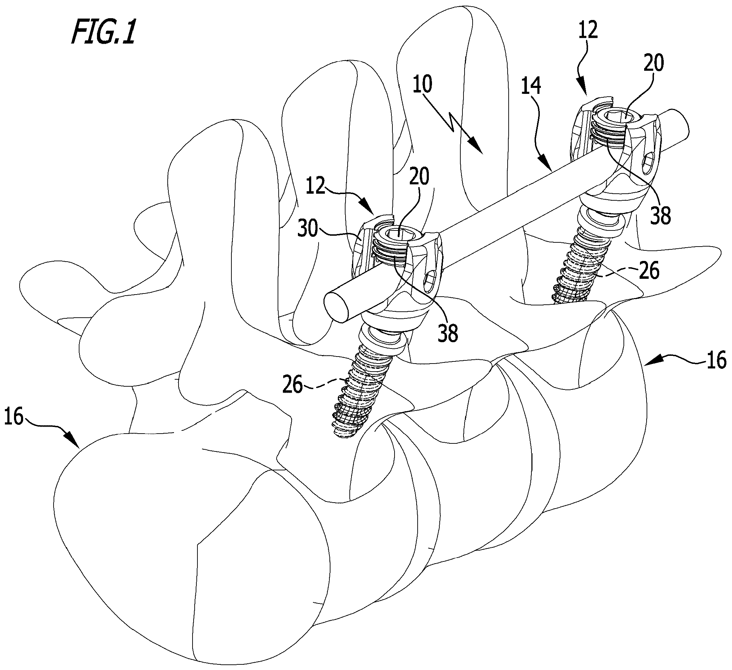

In Figur 1 ist beispielhaft ein insgesamt mit dem Bezugszeichen 10 bezeichnetes Wirbelsäulenstabilisierungssystem dargestellt, welches zwei Knochenschrauben 12 und ein an den beiden Knochenschrauben 12 festgelegtes Verbindungselement 14 umfasst. Die Knochenschrauben 12 sind an jeweils einem Wirbel 16 einer Wirbelsäule 18 festgelegt.

Selbstverständlich kann das Wirbelsäulenstabilisierungssystem 10 auch mehr als zwei Knochenschrauben 12 umfassen. Diese können beispielsweise über ein oder mehrere Verbindungselemente 14 miteinander verbunden werden.

In Figur 1 ist ein Verbindungselement 14 beispielhaft in Form eines Rundstabes dargestellt. Denkbar sind auch plattenförmige Verbindungselemente mit entsprechend ausgebildeten Abschnitten, die in Verbindungselementaufnahmen der Knochenschrauben 12 eingesetzt und beispielsweise mit jeweils einer Fixierschraube 20 festgelegt werden können.

Bei den Knochenschrauben 12 kann es sich grundsätzlich um herkömmliche, am Markt verfügbare Pedikelschrauben handeln. Vorzugsweise ist mindestens eine der Knochenschrauben 12 jedoch in Form eines Pedikelschraubensystems 22 ausgebildet, welches nachfolgend im Einzelnen näher erläutert wird.

Jedes der Pedikelschraubensysteme 22 umfasst eine Pedikelschraube 24 mit einem Schraubenschaft 26, welcher ein Außengewinde 28 aufweist, beispielsweise in Form eines selbstschneidenden Knochengewindes, und einen kugelgelenkig am Schraubenschaft 26 gelagerten Schraubenkopf 30. Der Schraubenkopf 30 weist eine zwischen zwei freien Schenkeln 32 gebildete Verbindungselementaufnahme 34 für das Verbindungselement 14 des Wirbelsäulenstabilisierungssystems 10 auf.

An der Verbindungselementaufnahme 34 ist ferner ein Innengewinde 36 vorgesehen, welches korrespondierend zu einem Außengewinde 38 der Fixierschraube 20 ausgebildet ist, sodass die Fixierschraube 20 zum Festlegen des Verbindungselements 14 ausgehend von freien Enden der Schenkel 32 in die Verbindungselementaufnahme 34 eingeschraubt werden kann, um das Verbindungselement 14 am Schraubenkopf 30 festzulegen.

Zur Ausbildung eines Kugelgelenks 40 zwischen dem Schraubenschaft 26 und dem Schraubenkopf 30 ist ein proximales Ende des Schraubenschafts 26 in Form eines Gelenkkopfs 42 ausgebildet mit einer in proximaler Richtung weisenden ebenen Endfläche 44, an welche eine einen Teil einer Kugeloberfläche bildende Gelenkkopffläche 46 angrenzt. In proximaler Richtung weisend ist im Gelenkkopf 42 eine Werkzeugelementaufnahme 48 ausgebildet, beispielsweise in Form eines Innenvielkant oder eines Innenvielrund.

Am Schraubenkopf 30 ist eine Gelenkkopfaufnahme 50 ausgebildet in Form eines korrespondierend zum Gelenkkopf 42 ausgebildeten Sitzes 52, welcher in eine in distaler Richtung sich im Innendurchmesser verjüngende Durchbrechung 54 mündet, die an einem distalen Ende 56 des Schraubenkopfs 30 ausgebildet ist und aus der der Schraubenschaft 26 distalseitig des Gelenkkopfs 42 vorsteht.

Distalseitig schließt sich an den Gelenkkopf 42 ein gewindefreier Schaftabschnitt 58 an, welcher distalseitig von einem Ringflansch 60 begrenzt ist. Der Ringflansch 60 weist einen Außendurchmesser auf, der etwas größer ist als ein Außendurchmesser des Gelenkkopfs 42.

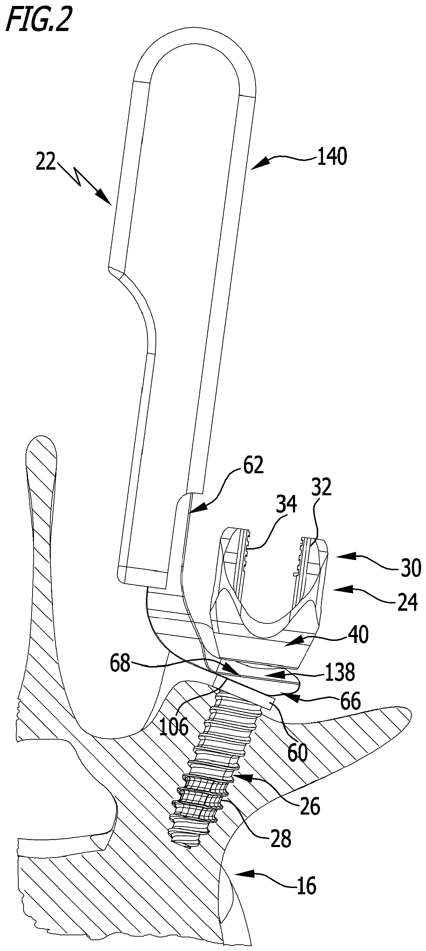

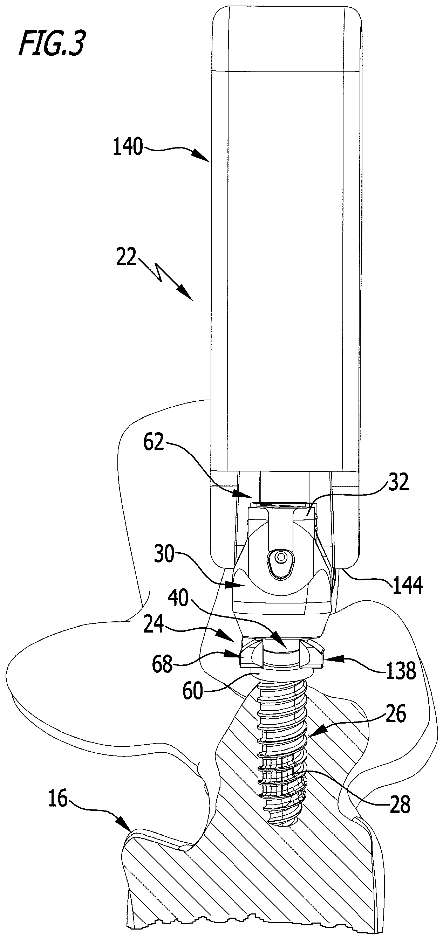

Das Pedikelschraubensystem 22 umfasst ferner eine Knochenausrichtvorrichtung 62 und eine Kopplungseinrichtung 64 zum kraft- und/oder formschlüssigen Koppeln der Knochenausrichtvorrichtung 62 und der Pedikelschraube 24 in einer Ausrichtstellung, wie sie beispielsweise in den Figuren 2 bis 7 beispielshaft dargestellt ist.

Die Knochenausrichtvorrichtung 62 umfasst eine Klemmplatte 66, welche einen Kopplungsabschnitt 68 der Knochenausrichtvorrichtung 62 bildet. Proximalseitig schließt sich an den Kopplungsabschnitt 68 ein Handhabungsabschnitt 70 an.

Der Kopplungsabschnitt 68 umfasst zwei im Wesentlichen parallel zueinander verlaufende, aus einer Grundstellung federnd aufeinander zu oder voneinander weg bewegbare Klemmschenkel 72, die durch einen Einführschlitz 74 voneinander getrennt sind. Der Einführschlitz 74 erstreckt sich teilweise in den Handhabungsabschnitt 70 hinein.

Ein proximales Ende der Knochenausrichtvorrichtung 62 bildet einen die beiden Klemmschenkel 72 miteinander verbindender Griffbereich 76 in Form einer Verbindungsplatte 78.

Eine Breite des Einführschlitzes 74 nimmt in Richtung auf freie Enden 80 der Klemmschenkel 72 hin zu, sodass Aufgleitflächen 82 ausgebildet werden, die in distaler Richtung weisend gegeneinander geneigt sind.

Die Kopplungsreinrichtung 64 umfasst erste und zweite Kopplungselemente 84 und 86, die in der Ausrichtstellung kraft- und/oder formschlüssig in Eingriff stehen. Sie sind einerseits an der Knochenausrichtvorrichtung 62 und andererseits an der Pedikelschraube 24 angeordnet und ausgebildet.

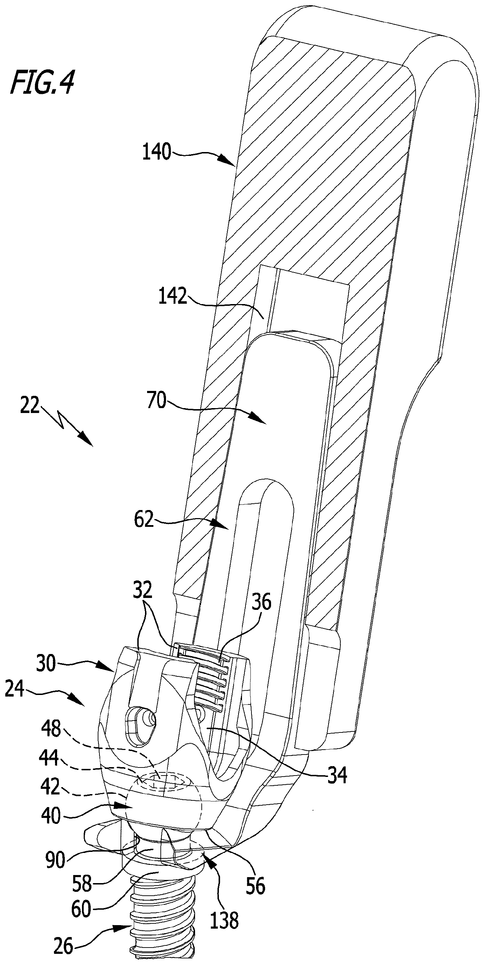

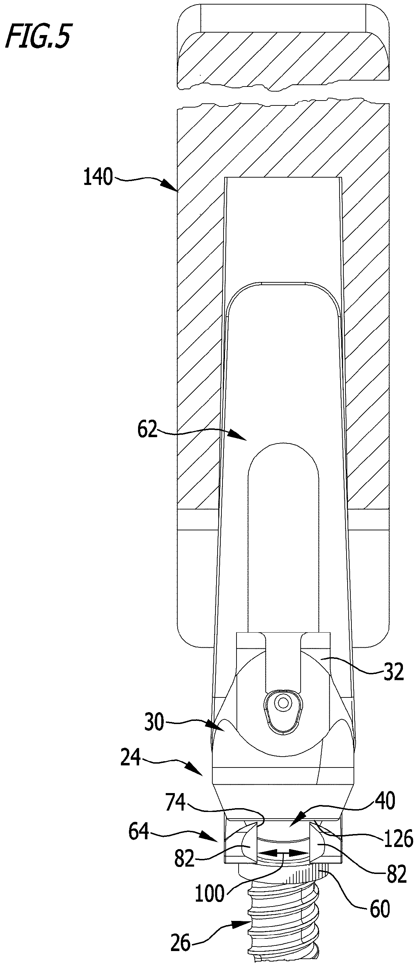

Bei dem in Figuren schematisch dargestellten Ausführungsbeispiel ist das erste Kopplungselement 84 in Form eines Kopplungsvorsprungs 88 ausgebildet, das zweite Kopplungselement 86 in Form einer zum Kopplungsvorsprung 88 korrespondierenden Kopplungsaufnahme 90. Wie insbesondere in Figuren 4 und 5 gut zu erkennen, greift der Kopplungsvorsprung 88 in der Ausrichtstellung formschlüssig oder im Wesentlichen formschlüssig in die Kopplungsaufnahme 90 ein.

Der Kopplungsvorsprung 88 ist einstückig mit dem Schraubenschaft 26 ausgebildet, und zwar in Form des gewindefreien Schaftabschnitts 58. Somit ist der Kopplungsvorsprung 88 zwischen dem Außengewinde 28 und dem Gelenkkopf 42 angeordnet.

Die Kopplungsaufnahme 90 ist in Form einer Durchbrechung 92 ausgebildet, und zwar als Bohrung 94. Ein Innendurchmesser 96 der Bohrung 94 ist an einen Außendurchmesser 98 des gewindefreien Schaftabschnitts 58 angepasst. Eine Breite 100 des Einführschlitzes 74 ist kleiner als der Außendurchmesser 98.

Die Kopplungsaufnahme 90 definiert eine Kopplungsaufnahmelängsachse 102. Diese verläuft senkrecht zu einer Ebene 104, die durch eine Unterseite 106 der Klemmplatte 66 definiert wird.

Der Kopplungsabschnitt 68 und der Handhabungsabschnitt 70 definieren jeweils Längsachsen 108 und 110, die gegeneinander um einen Winkel 112 abgewinkelt sind. Der Winkel 112 liegt vorzugsweise in einem Bereich von etwa 60° bis etwa 120° und kann insbesondere etwa 90° betragen.

Bei dem in den Figuren dargestellten Ausführungsbeispiel der Knochenausrichtvorrichtung 62 bildet die Kopplungsaufnahme 90 eine Schraubenschaftaufnahme 114, die der Schraubenschaft 26 in der Ausrichtstellung durchsetzt, also mindestens teilweise in diese eingreift.

Die Kopplungseinrichtung 64 umfasst ferner ein Anschlagelement 116, an welchem sich die Knochenausrichtvorrichtung 62 in der Ausrichtstellung einerseits abstützt. Die Unterseite 106 definiert eine Anschlagelementanlagefläche 118, die in der Ausrichtstellung am Anschlagelement 116 anliegt. Die Anschlagelementanlagefläche 118 ist bei dem in den Figuren dargestellten Ausführungsbeispiel eben ausgebildet.

Das Anschlagelement 116 weist eine ebene Anschlagfläche 120 auf, an welcher sich die Knochenausrichtvorrichtung 62 mit ihrer Anschlagelementanlagefläche 118 in der Ausrichtstellung abstützt.

Das Anschlagelement 116 ist am Schraubenschaft 26 angeordnet beziehungsweise ausgebildet, und zwar in Form des Ringflansches 60. Das Anschlagelement 116 ist vorzugsweise einstückig mit dem Schraubenschaft 26 ausgebildet. Bei alternativen Ausführungsformen kann der Schraubenschaft 26 mit dem Anschlagelement 116 jedoch auch kraft- und/oder stoffschlüssig verbunden sein, beispielsweise verpresst, verklebt, verlötet oder verschweißt.

Der Ringflansch 60 ist so bemessen, dass dessen Außendurchmesser 122 größer ist als ein maximaler Außendurchmesser 124 des Außengewindes 28. Zudem grenzt das Anschlagelement 116 direkt an das Außengewinde 28 an.

Ferner weist der Schraubenkopf 30 eine in Richtung auf das Außengewinde 28 hin weisende Anlagefläche 126 auf, an welcher sich die Knochenausrichtvorrichtung 62 in der Ausrichtstellung abstützt, und zwar mit einer Oberseite 128 der Klemmplatte 66 derselben. Die Anlagefläche 126 ist bei dem in den Figuren beispielhaft dargestellten Schraubenkopf 30 eben ausgebildet. Alternativ kann sie auch vom Schraubenkopf 30 in Richtung auf das Außengewinde 28 hin weisend konkav gekrümmt ausgebildet sein.

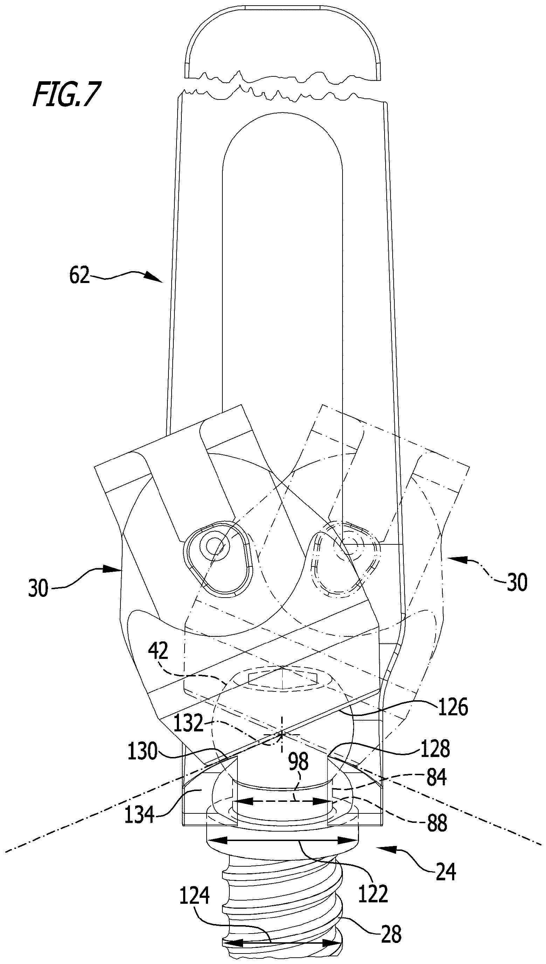

Die Oberseite 128 der Knochenausrichtvorrichtung 62 definiert eine Führungsfläche 130 zum Vorgeben einer Bewegungsrichtung für eine Relativbewegung zwischen dem Schraubenkopf 30 und dem Schraubenschaft 26. Wie beispielsweise in Figur 7 gut zu erkennen, liegt der Schraubenkopf 30 in der Ausrichtstellung an der Führungsfläche 130 an und ist an dieser um eine Schwenkachse 132 relativ zum Schraubenschaft 26 verschwenkbar geführt.

Die Führungsfläche 130 ist in Richtung auf den Schraubenkopf 30 hin konvex gekrümmt ausgebildet und kann insbesondere einen Teil einer Zylinderoberfläche 134 bilden, insbesondere einen Teil einer Oberfläche eines geraden Kreiszylinders.

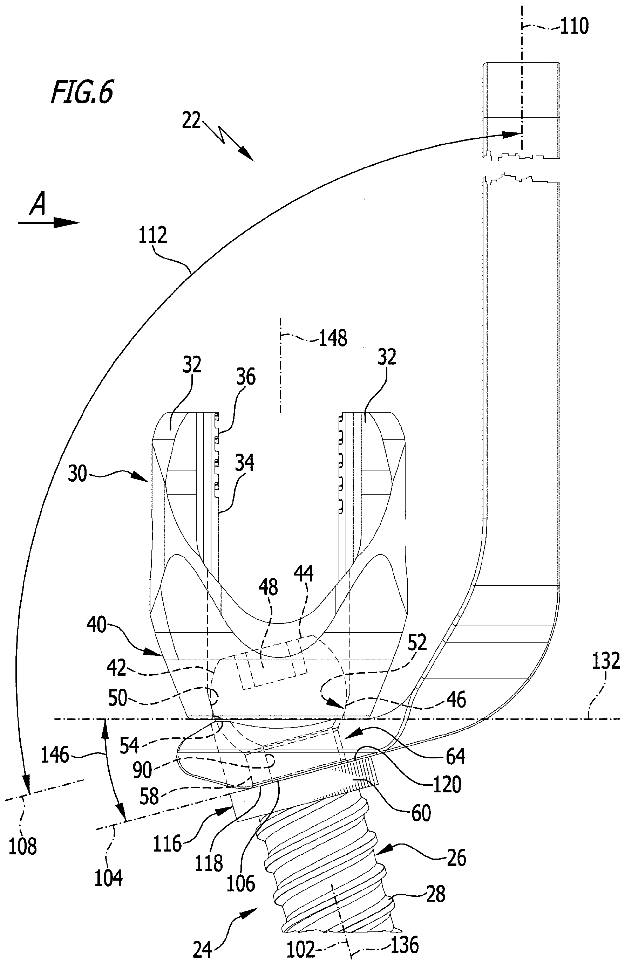

Die Schwenkachse 132 verläuft quer, bei dem in den Figuren dargestellten Ausführungsbeispiel senkrecht, zur Kopplungsaufnahmelängsachse 102. Damit, wie insbesondere in Figur 6 dargestellt, verläuft die Schwenkachse 132 auch senkrecht zu einer Schraubenschaftlängsachse 136 des Schraubenschafts 26.

Die Klemmplatte 66 bildet einen Führungskörper 138, welcher die Führungsfläche 130, die Anschlagelementanlagefläche 118 und das zweite Kopplungselement 86 umfasst.

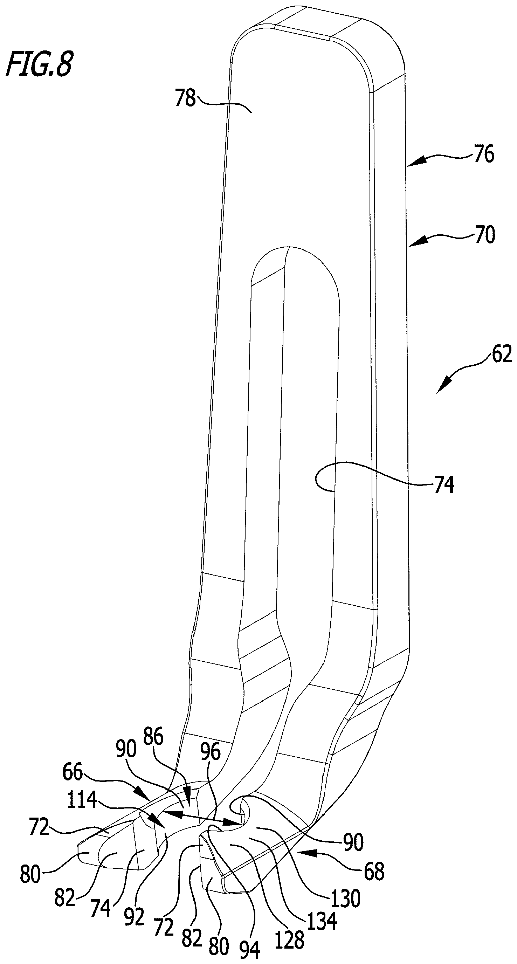

Die besondere Ausgestaltung der Knochenausrichtvorrichtung 62 ermöglicht es aufgrund der besonderen Anordnung und Ausbildung des Einführschlitzes 74, dass die Klemmschenkel 72 aus der in Figur 8 dargestellten Grundstellung aufeinander zu oder voneinander weg federnd bewegbar sind.

Zum Einführen des Kopplungsvorsprungs 88 in die Kopplungsaufnahme 90 durch den Einführschlitz 74 werden die Klemmschenkel 72 etwas aufgespreizt, sodass der Einführschlitz 74, welcher in der Grundstellung schmaler als der Außendurchmesser 98 ist, etwas aufgeweitet wird. Gleitet der Schaftabschnitt 58 an den Aufgleitflächen 82 auf, so weitet sich der Einführschlitz 74 und der Schaftabschnitt 58 kann durch diesen hindurch in die Kopplungsaufnahme 90 eingeschoben werden. Greift der Schaftabschnitt 58 in die Kopplungsaufnahme 90 ein, schnappen beziehungsweise federn die Klemmschenkel 72 aufeinander zu zurück und umschließen den Schaftabschnitt 58 teilweise. In der beschriebenen Weise lässt sich die Knochenausrichtvorrichtung 62, und zwar deren Kopplungsabschnitt 68, auf die Pedikelschraube 24 aufclipsen beziehungsweise aufrasten.

Die Führungsfläche 130 gestattet es aufgrund ihrer Ausgestaltung nur, den Schraubenkopf 30 ausschließlich um die Schwenkachse 132 relativ zum Schraubenschaft 26 zu verschwenken. Damit schränkt die Knochenausrichtvorrichtung 62 in der Ausrichtstellung die Beweglichkeit des Schraubenkopfs 30 und des Schraubenschafts 26 von ursprünglich drei Bewegungsfreiheitsgraden der Rotation, die durch das Kugelgelenk 40 definiert werden, auf lediglich einen einzigen Rotationsfreiheitsgrad ein, welcher durch die Schwenkachse 132 definiert wird. Es ist also nur noch die Verschwenkbewegung, wie in Figur 7 schematisch dargestellt, um die Schwenkachse 132 möglich. Damit wird die als Polyaxialschraube ausgebildete Pedikelschraube 24 im Zusammenwirken mit der Knochenausrichtvorrichtung 62 praktisch auf Funktionalität einer Monoaxialschraube reduziert, bei welcher der Schraubenkopf 30 relativ zum Schraubenschaft 26 nur um die eine Schwenkachse 132 verschwenkbar ist.

Um zu verhindern, dass sich die Knochenausrichtvorrichtung 62 unbeabsichtigt von der Pedikelschraube 24 löst, umfasst das Pedikelschraubensystem 22 optional eine Blockierhülse 140. Diese weist eine korrespondierend zum Handhabungsabschnitt 70 ausgebildete Aufnahme 142 auf, in die der Handhabungsabschnitt 70 parallel zur Längsachse 110 eingeschoben werden kann, wie dies beispielhaft in Figur 4 dargestellt ist.

Die Aufnahme 142 ist bis nahe an ein distales Ende 144 der Blockierhülse 140 heran allseitig geschlossen, sodass die Klemmschenkel 72 nicht mehr voneinander weg verschwenkt werden können. Die an der Pedikelschraube 24 gekoppelte Knochenausrichtvorrichtung 62 lässt sich dann nicht mehr von der Pedikelschraube 24 lösen, ohne dass die Blockierhülse 140 wieder in proximaler Richtung vom Handhabungsabschnitt 70 abgezogen wird.

Die Oberseite 128 des Führungskörpers 138 ist relativ zur Unterseite 106 bei dem in den Figuren dargestellten Ausführungsbeispiel um einen Ausrichtwinkel 146 geneigt. Der Ausrichtwinkel 146 gibt die Neigung einer Längsachse 148 des Schraubenkopfs 30 relativ zur Schraubenschaftlängsachse 136 vor. Optional kann das Pedikelschraubensystem 22 mehrere Knochenausrichtvorrichtungen 62 umfassen, die sich in der Ausgestaltung des Führungskörpers 138 derart unterscheiden, dass sie unterschiedliche Ausrichtwinkel 146 aufweisen. So kann das Pedikelschraubensystem 22 einen Satz von Knochenausrichtvorrichtungen 62 umfassen, die es einem Operateur ermöglichen, eine Ausrichtung der Längsachse 148 und der Schraubenschaftlängsachse 136 gezielt zu wählen und damit der temporär in der Ausrichtstellung eine Monoaxialschraube bildenden Pedikelschraube 24 eine Schwenkrichtung für den Schraubenkopf 30 vorzugeben.

Durch den beschriebenen Satz von Knochenausrichtvorrichtungen 62 mit unterschiedlichen Ausrichtwinkeln 146 wird es einem Operateur ermöglicht, eine intraoperative Einstellung der Schwenkachse 132 zu wählen. So kann ein Arzt durch entsprechende Wahl der Knochenausrichtvorrichtung 62 mit dem von ihm gewünschten Ausrichtwinkel 146 die Stellung des Schraubenkopfs 30 nach einer Rotation des Wirbels 16 mit Hilfe der Knochenausrichtvorrichtung 62 beeinflussen und in der Folge das Einsetzen des Verbindungselements 14 in die Verbindungselementaufnahme 34 der Pedikelschraube 24 vereinfachen.

Mit dem Pedikelschraubensystem 22 ist die teilweise, insbesondere richtungsabhängige, Blockierung der Polyaxialität der Pedikelschraube 24 durch die temporäre Kopplung mit der Knochenausrichtvorrichtung 62 in der Ausrichtstellung sowie optional das intraoperative Einstellen eines Winkels zwischen der Längsachse 148 und der Schraubenschaftlängsachse 136 durch entsprechende Wahl einer Knochenausrichtvorrichtung 62 mit gewünschtem Ausrichtwinkel 146 möglich. Trotzdem bleibt die Möglichkeit, den Schraubenkopf 30 relativ zum Schraubenschaft 26 um die Längsachse 148 zu rotieren, auch in der Ausrichtstellung erhalten.

Wird wieder die volle Polyaxialität der Pedikelschraube 24 gewünscht, wird in der beschriebenen Weise die Knochenausrichtvorrichtung 62 wieder von der Pedikelschraube 24 gelöst.

### Bezugszeichenliste

- 10: Wirbelsäulenstabilisierungssystem
- 12: Knochenschraube
- 14: Verbindungselement
- 16: Wirbel
- 18: Wirbelsäule
- 20: Fixierschraube
- 22: Pedikelschraubensystem
- 24: Pedikelschraube
- 26: Schraubenschaft
- 28: Außengewinde
- 30: Schraubenkopf
- 32: Schenkel
- 34: Verbindungselementaufnahme
- 36: Innengewinde
- 38: Außengewinde
- 40: Kugelgelenk
- 42: Gelenkkopf
- 44: Endfläche
- 46: Gelenkkopffläche
- 48: Werkzeugelementaufnahme
- 50: Gelenkkopfaufnahme
- 52: Sitz
- 54: Durchbrechung
- 56: Ende
- 58: Schaftabschnitt
- 60: Ringflansch
- 62: Knochenausrichtvorrichtung
- 64: Kopplungseinrichtung
- 66: Klemmplatte
- 68: Kopplungsabschnitt
- 70: Handhabungsabschnitt
- 72: Klemmschenkel
- 74: Einführschlitz
- 76: Griffbereich
- 78: Verbindungsplatte
- 80: Ende
- 82: Aufgleitfläche
- 84: erstes Kopplungselement
- 86: zweites Kopplungselement
- 88: Kopplungsvorsprung
- 90: Kopplungsaufnahme
- 92: Durchbrechung
- 94: Bohrung
- 96: Innendurchmesser
- 98: Außendurchmesser
- 100: Breite
- 102: Kopplungsaufnahmelängsachse
- 104: Ebene
- 106: Unterseite
- 108: Längsachse
- 110: Längsachse
- 112: Winkel
- 114: Schraubenschaftaufnahme
- 116: Anschlagelement
- 118: Anschlagelementanlagefläche
- 120: Anschlagfläche
- 122: Außendurchmesser
- 124: Außendurchmesser
- 126: Anlagefläche
- 128: Oberseite
- 130: Führungsfläche
- 132: Schwenkachse
- 134: Zylinderoberfläche
- 136: Schraubenschaftlängsachse
- 138: Führungskörper
- 140: Blockierhülse
- 142: Aufnahme
- 144: Ende
- 146: Ausrichtwinkel
- 148: Längsachse

## Patentansprüche

1. Pedikelschraubensystem (22) umfassend eine Pedikelschraube (24) mit einem Schraubenschaft (26), welcher ein Außengewinde (28) aufweist, und einem kugelgelenkig am Schraubenschaft (26) gelagerten Schraubenkopf (30), welcher Schraubenkopf (30) eine Verbindungselementaufnahme (34) für ein Verbindungselement (14) eines Wirbelsäulenstabilisierungssystems (10) umfasst, **gekennzeichnet durch** eine Knochenausrichtvorrichtung (62) und eine Kopplungseinrichtung (64) zum kraft- und/oder formschlüssigen Koppeln der Knochenausrichtvorrichtung (62) und der Pedikelschraube (24) in einer Ausrichtstellung, in welcher eine Beweglichkeit des Schraubenkopfs (30) und des Schraubenschafts (26) von drei Bewegungsfreiheitsgraden der Rotation des kugelgelenkig am Schraubenschaft (26) gelagerten Schraubenkopfs (30) um mindestens einen Rotationsfreiheitsgrad reduziert ist, nämlich von ursprünglich drei Bewegungsfreiheitsgraden der Rotation auf einen oder zwei Bewegungsfreiheitsgrade der Rotation, wobei der Schraubenkopf (30) eine in Richtung auf das Außengewinde (28) hin weisende Anlagefläche (126) aufweist, an welcher sich die Knochenausrichtvorrichtung (62) in der Ausrichtstellung abstützt.

2. Pedikelschraubensystem nach Anspruch 1, **dadurch gekennzeichnet, dass** die Knochenausrichtvorrichtung (62) in Form einer Klemmplatte (66) ausgebildet ist oder eine Klemmplatte (66) umfasst.

3. Pedikelschraubensystem nach einem der voranstehenden Ansprüche, **dadurch gekennzeichnet, dass** die Kopplungseinrichtung (64) erste und zweite Kopplungselemente (84, 86) umfasst, welche in der Ausrichtstellung kraft- und/oder formschlüssig in Eingriff stehen und einerseits an der Knochenausrichtvorrichtung (62) und andererseits an der Pedikelschraube (24) angeordnet oder ausgebildet sind.

4. Pedikelschraubensystem nach Anspruch 3, **dadurch gekennzeichnet, dass** die Knochenausrichtvorrichtung (62) einen Führungskörper (138) umfasst und dass der Führungskörper (138) die Führungsfläche (130), die Anschlagelementanlagefläche (118) und/oder eines der Kopplungselemente (86) der Kopplungseinrichtung (64) umfasst.

5. Pedikelschraubensystem nach Anspruch 3 oder 4, **dadurch gekennzeichnet, dass** die Knochenausrichtvorrichtung (62) einen Kopplungsabschnitt (68) und einen Handhabungsabschnitt (70) umfasst und dass der Kopplungsabschnitt (68) eines der Kopplungselemente (84) der Kopplungseinrichtung (64) umfasst.

6. Pedikelschraubensystem nach einem der Ansprüche 3 bis 5, **dadurch gekennzeichnet, dass** das erste Kopplungselement (84) in Form eines Kopplungsvorsprungs (88) und dass das zweite Kopplungselement (86) in Form einer zum Kopplungsvorsprung (88) korrespondierenden Kopplungsaufnahme (90) ausgebildet sind.

7. Pedikelschraubensystem nach Anspruch 6, **dadurch gekennzeichnet, dass**
a) der Kopplungsvorsprung (88) in der Ausrichtstellung formschlüssig oder im Wesentlichen formschlüssig in die Kopplungsaufnahme (90) eingreift
und/oder
b) der Kopplungsvorsprung (88) einstückig mit dem Schraubenschaft (26) ausgebildet ist
und/oder
c) der Kopplungsvorsprung (88) in Form eines gewindefreien Schaftabschnitts (58) des Schraubenschafts (26) ausgebildet ist.

8. Pedikelschraubensystem nach Anspruch 6 oder 7, **dadurch gekennzeichnet, dass** der Kopplungsvorsprung (88) in Form eines gewindefreien Schaftabschnitts (58) des Schraubenschafts (26) ausgebildet ist und dass ein Innendurchmesser (96) der Kopplungsaufnahme (90) an einen Außendurchmesser (98) des gewindefreien Schaftabschnitts (58) angepasst ist.

9. Pedikelschraubensystem nach einem der voranstehenden Ansprüche, **dadurch gekennzeichnet, dass** der Schraubenschaft (26) einen Gelenckopf (42) aufweist und dass der Schraubenkopf (30) eine zum Gelenckopf (42) korrespondierende Gelenkkopfaufnahme (50) zur Ausbildung eines Kugelgelenks (40) im Zusammenwirken mit dem Gelenkkopf (42) aufweist und dass insbesondere der Kopplungsvorsprung (88) zwischen dem Außengewinde (28) und dem Gelenkkopf (42) angeordnet ist.

10. Pedikelschraubensystem nach einem der voranstehenden Ansprüche, **dadurch gekennzeichnet, dass** die Kopplungseinrichtung (64) ein Anschlagelement (116) umfasst, an welchem sich die Knochenausrichtvorrichtung (62) in der Ausrichtstellung abstützt.

11. Pedikelschraubensystem nach Anspruch 10, **dadurch gekennzeichnet, dass** das Anschlagelement (116) einstückig mit dem Schraubenschaft (26) ausgebildet oder kraft- und/oder stoffschlüssig mit dem Schraubenschaft (26) verbunden ist, insbesondere verpresst, verklebt, verlötet oder verschweißt.

12. Pedikelschraubensystem nach einem der voranstehenden Ansprüche, **dadurch gekennzeichnet, dass** die Knochenausrichtvorrichtung (62) eine Führungsfläche (130) zum Vorgeben einer Bewegungsrichtung für eine Relativbewegung zwischen dem Schraubenkopf (30) und dem Schraubenschaft (26) aufweist und dass der Schraubenkopf (30) in der Ausrichtstellung an der Führungsfläche (130) anliegt und an dieser um eine Schwenkachse (132) relativ zum Schraubenschaft (26) verschwenkbar geführt ist.

13. Pedikelschraubensystem nach Anspruch 12, **dadurch gekennzeichnet, dass**,
a) das Pedikelschraubensystem mehrere Knochenausrichtvorrichtungen (62) umfasst, bei denen die Anschlagelementanlagefläche (118) und eine Führungsflächenlängsachse der Führungsfläche (130) unterschiedliche Ausrichtwinkel (146) relativ zueinander aufweisen,
und/oder
b) die Führungsfläche (130) einen Teil einer Zylinderoberfläche (134) bildet, insbesondere einen Teil einer Oberfläche eines geraden Kreiszylinders,
und/oder
c) die Schwenkachse (132) in der Ausrichtstellung quer, insbesondere senkrecht, zu einer Schraubenschaftlängsachse (136) des Schraubenschafts (26) verläuft.

14. Pedikelschraubensystem nach einem der voranstehenden Ansprüche, **dadurch gekennzeichnet, dass** die Knochenausrichtvorrichtung (62) eine Schraubenschaftaufnahme (114) aufweist, in die der Schraubenschaft (26) in der Ausrichtstellung mindestens teilweise eingreift.

15. Wirbelsäulenstabilisierungssystem (10) umfassend mindestens zwei Knochenschrauben (12) und mindestens ein an den mindestens zwei Knochenschrauben (12) festlegbares Verbindungselement (14), **dadurch gekennzeichnet, dass** mindestens eine der mindestens zwei Knochenschrauben (12) in Form eines Pedikelschraubensystems (22) nach einem der voranstehenden Ansprüche ausgebildet ist.

## Claims

1. Pedicle screw system (22), comprising a pedicle screw (24) having a screw shaft (26) with an external thread (28) and having a screw head (30) supported on the screw shaft (26) in a ball-and-socket joint relationship therewith, which screw head (30) comprises a connecting element receptacle (34) for a connecting element (14) of a spinal stabilization system (10), **characterized by** a bone alignment apparatus (62) and a coupling device (64) for force-locking coupling and/or form-locking coupling of the bone alignment apparatus (62) and the pedicle screw (24) in an alignment position in which a mobility of the screw head (30) and the screw shaft (26) is reduced from three degrees of freedom of movement in rotation of the screw head (30) supported on the screw shaft (26) in a ball-and-socket joint relationship therewith by at least one degree of freedom of rotation, namely from originally three degrees of freedom of movement in rotation to one or two degree(s) of freedom movement in rotation, wherein the screw head (30) has a contact face (126) pointing in a direction towards the external thread (28), on which contact face (126) the bone alignment apparatus (62) is supported when in the alignment position.

2. Pedicle screw system in accordance with claim 1, **characterized in that** the bone alignment apparatus (62) is configured in the form of a clamping plate (66) or comprises a clamping plate (66).

3. Pedicle screw system in accordance with any one of the preceding claims, **characterized in that** the coupling device (64) comprises first and second coupling elements (84, 86) which are in force-locking engagement and/or form-locking engagement when in the alignment position and are arranged or formed on the bone alignment apparatus (62) on the one hand and on the pedicle screw (24) on the other hand.

4. Pedicle screw system in accordance with claim 3, **characterized in that** the bone alignment apparatus (62) comprises a guide body (138) and **in that** the guide body (138) comprises the guide face (130), the stop element contact face (118) and/or one of the coupling elements (86) of the coupling device (64).

5. Pedicle screw system in accordance with claim 3 or 4, **characterized in that** the bone alignment apparatus (62) comprises a coupling section (68) and a manipulating section (70) and **in that** the coupling section (68) comprises one of the coupling elements (84) of the coupling device (64).

6. Pedicle screw system in accordance with any one of claims 3 to 5, **characterized in that** the first coupling element (84) is configured in the form of a coupling projection (88) and **in that** the second coupling element (86) is configured in the form of a coupling receptacle (90) corresponding to the coupling projection (88).

7. Pedicle screw system in accordance with claim 6, **characterized in that**
a) the coupling projection (88) in the alignment position engages in the coupling receptacle (90) in a form-locking manner or in a substantially form-locking manner
and/or
b) the coupling projection (88) is configured in one piece with the screw shaft (26)
and/or
c) the coupling projection (88) is configured in the form of a non-threaded shaft section (58) of the screw shaft (26).

8. Pedicle screw system in accordance with claim 6 or 7, **characterized in that** the coupling projection (88) is configured in the form of a non-threaded shaft section (58) of the screw shaft (26) and **in that** an internal diameter (96) of the coupling receptacle (90) is adapted to an external diameter (98) of the non-threaded shaft section (58).

9. Pedicle screw system in accordance with any one of the preceding claims, **characterized in that** the screw shaft (26) has a joint head (42) and **in that** the screw head (30) has a joint head receptacle (50) corresponding to the joint head (42) for forming a ball-and-socket joint (40) in cooperation with the joint head (42) and in particular **in that** the coupling projection (88) is arranged between the external thread (28) and the joint head (42).

10. Pedicle screw system in accordance with any one of the preceding claims, **characterized in that** the coupling device (64) comprises a stop element (116) on which the bone alignment apparatus (62) is supported when in the alignment position.

11. Pedicle screw system in accordance with claim 10, **characterized in that** the stop element (116) is configured in one piece with the screw shaft (26) or is connected to the screw shaft (26) in a force-locking manner and/or with a substance-to-substance bond, in particular by press-fitting, adhesive bonding, soldering or welding.

12. Pedicle screw system in accordance with any one of the preceding claims, **characterized in that** the bone alignment apparatus (62) has a guide face (130) for predetermining a moving direction for a relative movement between the screw head (30) and the screw shaft (26) and **in that** the screw head (30) in the alignment position is in contact against the guide face (130) and is guided thereon for pivotal movement relative to the screw shaft (26) about a pivotal axis (132).

13. Pedicle screw system in accordance with claim 12, **characterized in that**
a) the pedicle screw system comprises a plurality of bone alignment apparatuses (62) in which the stop element contact face (118) and a guide face longitudinal axis of the guide face (130) have different alignment angles (146) relative to one another,
and/or
b) the guide face (130) forms a part of a cylindrical surface (134), in particular a part of a surface of a right circular cylinder,
and/or
c) the pivotal axis (132) runs transversely, in particular perpendicularly, to a screw shaft longitudinal axis (136) of the screw shaft (26) when in the alignment position.

14. Pedicle screw system in accordance with any one of the preceding claims, **characterized in that** the bone alignment apparatus (62) has a screw shaft receptacle (114) in which the screw shaft (26) engages at least partially when in the alignment position.

15. Spinal stabilization system (10) comprising at least two bone screws (12) and at least one connecting element (14) fixable on the at least two bone screws (12), **characterized in that** at least one of the at least two bone screws (12) is configured in the form of a pedicle screw system (22) in accordance with any one of the preceding claims.

## Revendications

1. Système de vis pédiculaire (22) comprenant une vis pédiculaire (24) avec une tige de vis (26), laquelle présente un filetage extérieur (28) et une tête de vis (30) reposant contre la tige de vis (26) par une articulation à rotule, laquelle tête de vis (30) comprend un logement d'élément de liaison (34) pour un élément de liaison (14) d'un système de stabilisation de rachis (10), **caractérisé par** un dispositif d'alignement d'os (62) et un équipement de couplage (64) pour un couplage par liaison de force et/ou de forme du dispositif d'alignement d'os (62) et de la vis pédiculaire (24) dans une position d'alignement dans laquelle une mobilité de la tête de vis (30) et de la tige de vis (26) de trois degrés de liberté de mouvement de la rotation de la tête de vis (30) reposant par une articulation à rotule contre la tige de vis (26) est réduite d'au moins un degré de liberté de rotation, à savoir d'initialement trois degrés de liberté de mouvement de la rotation à un ou deux degrés de liberté de mouvement de la rotation, dans lequel la tête de vis (30) présente une surface d'appui (126) pointant dans une direction allant vers le filetage extérieur (28), contre laquelle le dispositif d'alignement d'os (62) s'arc-boute dans la position d'alignement.

2. Système de vis pédiculaire selon la revendication 1, **caractérisé en ce que** le dispositif d'alignement d'os (62) est conçu sous la forme d'une plaque de serrage (66) ou comprend une plaque de serrage (66).

3. Système de vis pédiculaire selon l'une des revendications précédentes, **caractérisé en ce que** l'équipement de couplage (64) comprend des premiers et seconds éléments de couplage (84, 86), lesquels sont en prise par liaison de force et/ou de forme dans la position d'alignement et sont agencés ou conçus d'un côté contre le dispositif d'alignement d'os (62) et d'un autre côté contre la vis pédiculaire (24).

4. Système de vis pédiculaire selon la revendication 3, **caractérisé en ce que** le dispositif d'alignement d'os (62) comprend un corps de guidage (138) et **en ce que** le corps de guidage (138) comprend la surface de guidage (130), la surface d'appui d'élément de butée (118) et/ou l'un des éléments de couplage (86) de l'équipement de couplage (64).

5. Système de vis pédiculaire selon la revendication 3 ou 4, **caractérisé en ce que** le dispositif d'alignement d'os (62) comprend une section de couplage (68) et une section de manipulation (70) et **en ce que** la section de couplage (68) comprend l'un des éléments de couplage (84) de l'équipement de couplage (64).

6. Système de vis pédiculaire selon l'une des revendications 3 à 5, **caractérisé en ce que** le premier élément de couplage (84) est conçu sous la forme d'une saillie de couplage (88) et **en ce que** le second élément de couplage (86) est conçu sous la forme d'un logement de couplage (90) correspondant à la saillie de couplage (88).

7. Système de vis pédiculaire selon la revendication 6, **caractérisé en ce que**
a) la saillie de couplage (88) vient en prise dans le logement de couplage (90) par liaison de forme ou essentiellement par liaison de forme dans la position d'alignement
et/ou
b) la saillie de couplage (88) est conçue d'une seule pièce avec la tige de vis (26)
et/ou
c) la saillie de couplage (88) est conçue sous la forme d'une section de tige (58) sans filetage de la tige de vis (26).

8. Système de vis pédiculaire selon la revendication 6 ou 7, **caractérisé en ce que** la saillie de couplage (88) est conçue sous la forme d'une section de tige (58) sans filetage de la tige de vis (26) et **en ce qu'**un diamètre intérieur (96) du logement de couplage (90) est adapté à un diamètre extérieur (98) de la section de tige (58) sans filetage.

9. Système de vis pédiculaire selon l'une des revendications précédentes, **caractérisé en ce que** la tige de vis (26) présente une tête d'articulation (42) et **en ce que** la tête de vis (30) présente un logement de tête d'articulation (50) correspondant à la tête d'articulation (42) pour concevoir une articulation à rotule (40) en coopération avec la tête d'articulation (42) et **en ce qu'**en particulier la saillie de couplage (88) est agencée entre le filetage extérieur (28) et la tête d'articulation (42).

10. Système de vis pédiculaire selon l'une des revendications précédentes, **caractérisé en ce que** l'équipement de couplage (64) comprend un élément de butée (116) contre lequel le dispositif d'alignement d'os (62) s'arc-boute dans la position d'alignement.

11. Système de vis pédiculaire selon la revendication 10, **caractérisé en ce que** l'élément de butée (116) est conçu d'une seule pièce avec la tige de vis (26) ou relié à la tige de vis (26) par liaison de force et/ou de matériau, en particulier pressé, collé, brasé ou soudé.

12. Système de vis pédiculaire selon l'une des revendications précédentes, **caractérisé en ce que** le dispositif d'alignement d'os (62) présente une surface de guidage (130) pour préétablir une direction de mouvement pour un mouvement relatif entre la tête de vis (30) et la tige de vis (26) et **en ce que** la tête de vis (30) repose contre la surface de guidage (130) dans la position d'alignement et est guidée de façon pivotante contre celle-ci autour d'un axe de pivotement (132) par rapport à la tige de vis (26).

13. Système de vis pédiculaire selon la revendication 12, **caractérisé en ce que**
a) le système de vis pédiculaire comprend plusieurs dispositifs d'alignement d'os (62), où les surfaces d'appui d'élément de butée (118) et un axe longitudinal de surface de guidage de la surface de guidage (130) présentent des angles d'alignement (146) différents les uns des autres,
et/ou
b) la surface de guidage (130) forme une partie d'une surface de cylindre (134), en particulier une partie d'une surface d'un cylindre circulaire droit,
et/ou
c) l'axe de pivotement (132) dans la position d'alignement se déroule transversalement, en particulier perpendiculairement, à un axe longitudinal de tige de vis (136) de la tige de vis (26).

14. Système de vis pédiculaire selon l'une des revendications précédentes, **caractérisé en ce que** le dispositif d'alignement d'os (62) présente un logement de tige de vis (114) dans lequel la tige de vis (26) vient au moins partiellement en prise dans la position d'alignement.

15. Système de stabilisation de rachis (10) comprenant au moins deux vis à os (12) et au moins un élément de liaison (14) pouvant être fixé contre les au moins deux vis à os (12), **caractérisé en ce qu'**au moins une des au moins deux vis à os (12) est conçue sous la forme d'un système de vis pédiculaire (22) selon l'une des revendications précédentes.
